# EUROPÄISCHE PATENTSCHRIFT

(11) **EP 1 621 885 B1**
(45) Veröffentlichungstag und Bekanntmachung des Hinweises auf die Patenterteilung: **01.02.2012**
(21) Anmeldenummer: 05016248.6
(22) Anmeldetag: 26.07.2005
(51) Int. Cl.: G01N 33/487, G01N 21/86

(54) **Analysesystem mit Testelementhalter**
Analysis system with test element holder
Système d'analyse avec un support pour des élements analytiques

(30) Priorität: 28.07.2004 DE 102004036474
(43) Veröffentlichungstag der Anmeldung: 01.02.2006
(73) Patentinhaber: F.Hoffmann-La Roche AG, 4070 Basel (CH); Roche Diagnostics GmbH, 68305 Mannheim (DE)
(72) Erfinder: Jansen, Paul, 68163 Mannheim (DE); Ringelspacher, Yvonne, 67165 Waldsee (DE); Schulat, Jochen, 68305 Mannheim (DE)
(74) Vertreter: Stößel, Matthias

(56) Entgegenhaltungen:
- EP-A1- 1 256 798
- EP-A1- 1 382 968
- DE-C2- 19 781 162
- US-A- 5 795 543

## Beschreibung

Die vorliegende Erfindung betrifft ein Analysesystem zur Analyse einer Probe auf einem Testelement, das während der Analyse ausschließlich an einem äußeren Bereich gehalten und geführt wird und bei dem dabei ein innerer Bereich freiliegend bleibt.

Zur Analyse von Proben, beispielsweise Körperflüssigkeiten wie Blut oder Urin, werden häufig Analysesysteme verwendet, bei denen sich die zu analysierenden Proben auf einem Testelement befinden und in einem Testfeld gegebenenfalls mit einer oder mehreren Reagenzien auf dem Testelement reagieren, bevor sie analysiert werden. Die optische, insbesondere photometrische Auswertung von Testelementen stellt eines der gebräuchlichsten Verfahren zur schnellen Bestimmung der Konzentration von Analyten in Proben dar. Photometrische Auswertungen werden allgemein im Bereich der Analytik, der Umweltanalytik und vor allem im Bereich der medizinischen Diagnostik eingesetzt. Insbesondere im Bereich der Blutglukosediagnostik aus Kapillarblut besitzen Testelemente, die photometrisch ausgewertet werden, einen großen Stellenwert.

Es gibt verschiedene Formen von Testelementen. Bekannt sind z.B. im Wesentlichen quadratische Blättchen, die auch als Slides bezeichnet werden, in deren Mitte sich ein mehrschichtiges Testfeld befindet. Diagnostische Testelemente, die streifenförmig ausgebildet sind, werden als Teststreifen bezeichnet. Im Stand der Technik sind Testelemente umfassend beschrieben, beispielsweise in den Dokumenten EP 1256798, US 5795543, DE-A 197 53 847, EP-A 0 821 233, EP-A 0 821 234 oder WO97/02487. Die vorliegende Erfindung bezieht sich auf streifenförmige Testelemente.

Im Stand der Technik sind Testelemente bekannt, bei denen eine Probe auf eine Probenaufgabestelle aufgegeben wird und mittels Kapillarkraft an eine von der Probenaufgabestelle getrennte Detektionszone (Testfeld) transportiert wird. Ein solches Testelement ist beispielsweise Gegenstand der DE 197 53 847 A1. Darin wird ein analytisches Testelement zur Bestimmung eines Analyten in einer Flüssigkeit beschrieben. Es enthält einen inerten Träger, ein Nachweiselement und einen zum kapillaren Flüssigkeitstransport befähigten Kanal, der eine Probenaufgabeöffnung an einem und einer Entlüftungsöffnung am anderen Ende des zum kapillaren Flüssigkeitstransport befähigten Kanals besitzt. Der zum kapillaren Flüssigkeitstransport befähigte Kanal wird zumindest teilweise vom Träger und dem Nachweiselement gebildet und reicht in Richtung des kapillaren Transports von der Probenaufgabeöffnung zumindest bis zu der der Entlüftungsöffnung nächstgelegenen Kante des Nachweiselements, wobei sich eine Aussparung in einer den zum kapillaren Flüssigkeitstransport befähigten Kanal bildenden Fläche, an der die Probenaufgabeöffnung bildenden Kante des Testelements befindet. Die die Probenaufgabeöffnung bildende Kante des Testelements ist so auf einer Seite zumindest teilweise unterbrochen und die der Aussparung gegenüber liegende Fläche liegt frei. Die Aussparung in einer den kapillaren Kanal bildenden Fläche an der Kante des Testelements dient dazu, sicherzustellen, dass die Probenflüssigkeit in den kapillaren Kanal eintreten kann. Dies wird dadurch erreicht, dass der Probentropfen an der durch die Aussparung unterbrochenen Kante des Testelements, die der Probenaufgabeöffnung am nächsten liegt, direkt mit einer der Flächen in Kontakt gebracht werden kann, die in ihrer Verlängerung die innere Oberfläche der Kapillare bilden. Durch geeignete Wahl der Geometrie und Dimensionen der Aussparung wird erreicht, dass der Flüssigkeitstropfen unabhängig von der genauen Position der Dosierung mit sehr hoher Wahrscheinlichkeit mit der kapillaraktiven Zone in Kontakt kommt und bereitwillig in das Innere der Kapillare eingesaugt wird.

Zur analytischen Untersuchung einer Probe auf einem Testelement sind im Stand der Technik Testelement-Analysesysteme bekannt, die eine Testelementhalterung zum Positionieren des Testelements in einer Messposition und eine Mess- und Auswerteeinrichtung zur Durchführung einer Messung und Ermittlung eines hierauf basierenden Analyseresultats enthalten. WO 00/19185 A1 bezieht sich auf eine Vorrichtung zur photometrischen Auswertung von Testelementen, beinhaltend
- eine Beleuchtungseinheit mit mindestens einer ersten und einer zweiten Lichtquelle,
- eine Halterung zur Aufnahme eines Testelements mit einer Nachweiszone in der Weise, dass die Nachweiszone gegenüber der Beleuchtungseinheit positioniert wird,
- eine Detektionseinheit mit mindestens einem Detektor, der von der Nachweiszone reflektiert wird oder durch die Nachweiszone transmittiertes Licht detektiert,
- eine Steuerungseinheit, die die beiden Lichtquellen aktiviert und das von der Detektionseinheit generierte Signal als Detektionssignal aufnimmt und
- eine Auswerteeinheit, die die Detektionssignale auswertet, um die in der Probe enthaltene Analytkonzentration zu ermitteln.

Bekannte Messgeräte besitzen eine Öffnung, im Allgemeinen einen Schlitz, in den Testelemente eingeschoben werden können. Führungselemente stellen sicher, dass ein Testelement in der vorgesehenen Orientierung eingeschoben wird. Falls das Testelement manuell in das Gerät eingeführt wird, müssen konstruktive Merkmale des Geräts vorliegen, welche die gewünschte Positionierung des Testelements gewährleisten. Üblicherweise wird dies durch eine Begrenzung realisiert, die ein Einschieben über eine vorgegebene Zielposition verhindert. Ferner sind im Stand der Technik Analysesysteme bekannt, die ein Vorratsbehältnis (Magazin) mit einer Vielzahl von Testelementen enthalten. Dabei wird ein Testelement zum Beispiel mit einem Schieber oder Stößel aus dem Vorratsbehältnis an den Ort der Messung transportiert und nach der Durchführung der Messung automatisch aus dem Analysesystem ausgeworfen.

Aus DE 199 02 601 A1 ist eine Vorrichtung zum Entnehmen eines analytischen Verbrauchsmittels, insbesondere eines Testelements aus einem Vorratsbehältnis bekannt, das eine oder mehrere Kammern aufweist, die Verbrauchsmittel enthalten. Die Kammern weisen jeweils eine Entnahmeöffnung zum Entnehmen eines Verbrauchsmittels und eine der Entnahmeöffnung gegenüber liegende Einschuböffnung zum Einführen eines Stößels für den Transport des Verbrauchsmittels auf. Die Entnahmeöffnung und die Einschuböffnung sind zur Lagerung des Verbrauchsmittels mit einer Folie verschlossen. Die Vorrichtung umfasst einen Stößel, der zur Entnahme eines Verbrauchsmittels mittels einer Antriebseinheit verfahrbar ist.

Das Auswerfen der verbrauchten Testelemente birgt eine Kontaminations- bzw. Infektionsgefahr, da sie unkontrolliert in die Umwelt abgegeben werden und Reste des Probenmaterials (z.B. Blut, Urin oder interstitielle Flüssigkeit) auf ihrer Oberfläche tragen. Durch einen Rücktransport in ein in dem verwendeten Analysesystem enthaltenes Vorratsmagazin (Remagazinierung) oder einen Transport in ein zur Entsorgung der Testelemente vorgesehenes Wastemagazin könnte eine hygienische Handhabung und Entsorgung der Testelemente sichergestellt werden.

Bei den im Stand der Technik bekannten Testelement-Analysesystemen liegt das Testelement in der Messposition mit zumindest einem großen Anteil seiner Unterseite auf einer Messgerätoberfläche in dem Analysesystem auf. Die Unterseite wird beim Transport des Testelements in die und aus der Messposition über die Messgerätoberfläche geschoben. Die Führung des Testelements erfolgt dabei mittels seitlicher, zur Messgerätoberfläche senkrechter Führungsflächen. Bei einem System zur photometrischen Auswertung des Testelements enthält die Messgerätoberfläche üblicherweise ein Optikfenster, unter dem sich die Optik befindet. Das Aufliegen des Testelements mit einem großen Anteil seiner Unterseite auf der Messgerätoberfläche hat den Nachteil, dass eine flüssige Probe, die in der Umgebung einer Seitenkante des Testelements auf dieses aufgegeben wird, die Messgerätoberfläche verschmutzen kann. Beispielsweise kann ein Teil der flüssigen Probe durch Kapillarkräfte zwischen das Testelement und die Messgerätoberfläche gezogen werden, so dass ein weiter Bereich mit Probe benetzt wird, einschließlich des Optikfensters. Eine solche Verschmutzung tritt insbesondere auf, wenn das Testelement nach Durchführung der Messung über die Messgerätoberfläche in das Magazin zurückgezogen wird (Remagazinierung). Dabei wird an der zum Probenauftrag verwendeten Kante des Testelements haftende Probe auf die Messgerätoberfläche abgestreift.

Ein weiterer Nachteil der im Stand der Technik bekannten Analysesysteme ist, dass das Optikfenster versenkt in die Messgerätoberfläche eingebaut werden muss, um es vor einer Beschädigung durch die Reibung des Testelements zu schützen.

Aufgabe der vorliegenden Erfindung ist es daher, die genannten Nachteile des Standes der Technik zu vermeiden. Insbesondere soll bei dem Analysesystem beim Remagazinieren eines Testelements nach der Messung eine Verschmutzung von Oberflächen in dem Analysesystem mit Probe vermieden werden.

Diese Aufgabe wird erfindungsgemäß gelöst durch ein Analysesystem zur Analyse einer Probe auf einem Testelement, umfassend die Merkmale des Anspruchs 1. Vorzugsweise sind dabei die Analyseeinheit und die Detektionseinheit Teile einer Messoptik, die zur photometrischen Auswertung des Testelements dient.

Als Analyseeinheit dienen zum Beispiel bei der photometrischen Auswertung eine Lichtquelle und eine Optik und als Detektionseinheit z.B. ein Photodetektor, der das von dem mit Probe versorgten Testfeld reflektierte oder durch das Testfeld transmittierte Licht (optisches Signal) detektiert. Ein derartiges Detektionssignal wird in bekannter Weise zur Ermittlung der Analytkonzentration ausgewertet.

Eine Verschmutzung des Analysesystems mit Probe wird bei der vorliegenden Erfindung dadurch vermieden, dass das Testelement nur in einem äußeren Bereich geführt und gehalten wird und ein innerer Bereich des in den Testelementhalter eingeführten Testelements freiliegend bleibt. Unter dem inneren Bereich ist dabei der mittlere Teil der beiden Oberflächen des Testelements zu verstehen. Der Probenaufgabeort, an dem die Probe auf das Testelement gegeben wird, befindet sich dabei in dem inneren Bereich des Testelements, so dass er nicht in Kontakt mit dem Testelementhalter kommt und ihn nicht mit Probe kontaminieren kann. Das Führungselement ist in dem Analysesystem so angeordnet, dass ein in das Führungselement eingeführtes Testelement relativ zu der Analyseeinheit und zu der Detektionseinheit positioniert wird. Um z.B. eine exakte photometrische Auswertung durchführen zu können, ist eine präzise Positionierung des Testfeldes relativ zu der Messoptik notwendig.

Der Testelementhalter übernimmt bei der vorliegenden Erfindung vorzugsweise nicht nur die Funktion der Führung eines eingeführten Testelements, sondern auch dessen Halterung, damit es während der Messung in der Messposition bleibt. Das Testelement ist in den Testelementhalter reversibel einführbar, so dass es nach der Messung in der der Einführrichtung entgegengesetzten Richtung aus dem Testelementhalter entfernt werden kann.

Bei den in dem erfindungsgemäßen Analysesystem verwendeten Testelementen handelt es sich vorzugsweise um Teststreifen, in denen eine flüssige Probe, insbesondere Blut, Urin oder interstitielle Flüssigkeit, mittels Kapillarkraft vom Probenaufgabeort zum Testfeld transportiert wird. Ein zum kapillaren Flüssigkeitstransport geeigneter Kanal weist üblicherweise eine Eingangsöffnung und eine Entlüftungsöffnung auf. Vorzugsweise ist die Eingangsöffnung bei der vorliegenden Erfindung in der Nähe des Probenaufgabeortes, also im inneren Bereich des Testelements angeordnet. Die Entlüftungsöffnung ist bei der vorliegenden Erfindung vorzugsweise ebenfalls im inneren Bereich des Testelements angeordnet, so dass unbeabsichtigt aus der Entlüftungsöffnung austretende Probenflüssigkeit keine Kontamination des erfindungsgemäßen Testelementhalters verursachen kann.

In einer bevorzugten Ausführungsform enthält das Führungselement Trageflächen, auf denen das Testelement mit Auflageflächen in seinem äußeren Bereich aufliegt und Führungsflächen, an denen Seitenflächen des Testelements entlanggeführt werden. Es ist dabei darauf zu achten, dass noch genügend Spiel zwischen den Seitenflächen des Testelements und den Führungsflächen bleibt, um das Testelement mit minimalem Kraftaufwand in dem Führungselement bewegen zu können und den Verschleiß (z.B. Abrieb vom Testelement oder Kerbenbildung in den Führungswänden) gering zu halten.

In einer bevorzugten Ausführungsform der vorliegenden Erfindung sind die Führungsflächen schräg in Bezug auf die Seitenflächen angeordnet. Dadurch wird erreicht, dass ein Testelement beim Einführen in das Führungselement die Führungsflächen nicht über die gesamten Seitenflächen berührt, sondern nur mit einer Kante an den Führungsflächen entlanggeschoben wird. Dies ist insbesondere bei Testelementen von Vorteil, die aus verschiedenen, miteinander verklebten Schichten aufgebaut sind. Ein solches Testelement ist beispielsweise in DE 199 12 365 A1 beschrieben. Durch die Schräge der Führungsflächen werden diese nicht durch gegebenenfalls an den Seitenflächen des Testelements auftretenden Klebstoff verschmutzt.

Die Auflageflächen, auf denen das Testelement in seinem äußeren Bereich aufliegt, weisen vorzugsweise eine Breite von 0,1 mm bis 1 mm, besonders bevorzugt von 0,3 mm bis 0,5 mm auf. Damit sind sie bei entsprechend geringem Spiel ausreichend breit, um ein unerwünschtes Herausfallen des Testelements aus dem Führungselement zu verhindern.

Erfindungsgemäß enthält das Führungselement zwei sich gegenüber liegende Nuten, in die das Testelement mit seinem äußeren Bereich eingeschoben werden kann. Das Testelement wird in solch einem Führungselement in die zwei Nuten, je einer auf der linken und der rechten Seite des Testelements, in einer Schiebebewegung eingeführt. Die Nuten umschließen die Kanten und Seitenflächen des Testelements derart, dass das Testelement weder nach noch nach unten aus dem Führungselement herausfallen kann (geschlossene Führung).

Das Führungselement ist bei der vorliegenden Erfindung vorzugsweise oberhalb der Analyseeinheit und/oder der Detektionseinheit in dem Analysesystem angeordnet. Beispielsweise ist die Messoptik (inklusive Lichtquelle und Photodetektor) bei der photometrischen Auswertung mit etwas Abstand unterhalb des Führungselements in dem Analysesystem angeordnet. Es ist jedoch auch möglich, die Analyseeinheit oberhalb und die Detektionseinheit unterhalb des Führungselements anzuordnen oder umgekehrt.

Bei einer Ausführungsform eines erfindungsgemäßen Analysesystems ist das Führungselement so angeordnet, dass ein in das Führungselement eingeführtes Testelement in jeder Position mindestens einen Abstand von 1 mm zu der Analyseeinheit und zu der Detektionseinheit aufweist. Durch diesen Abstand wird gewährleistet, dass die flüssige Probe nicht durch Kapillarkräfte zwischen das Testelement und die Analyseeinheit oder die Detektionseinheit gezogen wird und diese verschmutzt. Ferner muss z.B. ein Optikfenster nicht mehr versenkt in die Messgerätoberfläche eingebaut werden, da dieses durch den Abstand vor einer mechanischen Belastung geschützt wird, so dass sich das Design des Analysesystems vereinfacht.

In einer bevorzugten Ausführungsform der vorliegenden Erfindung enthält der Testelementhalter einen Anschlag, gegen den ein Testelement beim Einführen in das Führungselement anschlägt, wobei eine definierte Position des Testelements in dem Testelementhalter erreicht wird. Durch den Anschlag wird festgelegt, wie weit das Testelement in das Führungselement eingeführt werden soll.

In einer besonders bevorzugten Ausführungsform der vorliegenden Erfindung umfasst das Testelement an einem Ende in dem inneren Bereich einen Probenaufgabeort, wobei das Testelement im Bereich des Probenaufgabeorts verjüngt ist. Die Verjüngung kann dabei beispielsweise die Form einer Schulter oder eine Rückschnitts haben. Sie hat den Vorteil, dass das Testelement nur über die Breite des verjüngten Endes in seinem inneren Bereich und nicht über die gesamte Breite mit Probe benetzt wird. Der äußere Bereich des Testelements, in dem es gehalten und geführt wird, bleibt so frei von Probe und eine Verschmutzung des mit dem äußeren Bereich in Kontakt stehenden Führungselements wird weitgehend vermieden. Ferner kann der verjüngte Bereich des Testelements mit dem Probenaufgabeort aus dem Testelementhalter herausragen, um die Probenaufnahme von außen zu ermöglichen. Insbesondere kann das Führungselement dabei einen Anschlag aufweisen, an dem der an den verjüngten Bereich anschließende breite (normale) Bereich des Testelements anschlägt, sobald es ausreichend weit in das Führungselement eingeführt ist.

Vorzugsweise umfasst das erfindungsgemäße Analysesystem des weiteren einen Vorratsbehälter für eine Vielzahl von Testelementen, in den die Testelemente nach Gebrauch aus dem Testelementhalter zurücktransportiert werden. Dies hat den Vorteil, dass die Handhabung und Entsorgung verbrauchter Testelemente in einer hygienischen Art und Weise erfolgt. Dabei weist das erfindungsgemäße Analysesystem vorzugsweise eine Transporteinrichtung zur automatischen Entnahme eines Testelements aus dem Vorratsbehälter, zum automatischen Transport des Testelements in den Testelementhalter und zum automatischen Rücktransport des Testelements nach Gebrauch in den Vorratsbehälter auf.

Die Transporteinrichtung umfasst beispielsweise einen Stößel, einen Haken oder eine Klammer, die an ein Testelement ankoppeln und dieses anschließend in eine gewünschte Position in dem Analysesystem transportieren können. In einer bevorzugten Ausführungsform der vorliegenden Erfindung weist das Testelement ein Testfeld auf, in dem die Probe analysiert wird, und das in dem inneren Bereich des Testelements positioniert ist. Zur qualitativen und quantitativen analytischen Bestimmung von Bestandteilen einer flüssigen Probe, insbesondere von Körperflüssigkeiten, sind in dem Testfeld Reagenzien eingebettet. Das Testfeld wird mit der Probe in Kontakt gebracht und die Reaktion von flüssiger Probe und Reagenzien führt bei Anwesenheit eines Zielanalyten zu einem nachweisbaren Signal, z.B. zu einem Farbumschlag, welches mit Hilfe der Analyseeinheit und der Detektionseinheit detektiert werden kann. Vorzugsweise enthält das Testelement bei der vorliegenden Erfindung eine Kapillare zum Leiten der Probe zu dem Testfeld.

In einer Ausführungsform der vorliegenden Erfindung ist der Testelementhalter mindestens zweiteilig, wobei ein in den Testelementhalter eingeführtes Testelement in seinem äußeren Bereich auf einem unteren Teil des Testelementhalters aufliegt und ein separater oberer Teil des Testelementhalters auf dem Testelement in seinem äußeren Bereich aufliegt. Ohne Testelement liegen die beiden Teile lose übereinander und werden seitlich gegen ein Verschieben gesichert. Wenn ein Testelement in das Führungselement zwischen den beiden Teilen des Testelementhalters eingeführt wird, werden die beiden Teile durch das Testelement auseinandergedrückt. Das Testelement passt daher spielfrei in das Führungselement und wird durch den oberen Teil, der auf seinem äußeren Bereich aufliegt, in Position gehalten, z.B. wenn die Probenaufnahme- und/oder die Messposition erreicht ist.

Vorzugsweise ist an dem oberen Teil des Testelementhalters mindestens eine Druckfeder angeordnet, die auf den oberen Teil eine Kraft in Richtung des unteren Teils des Testelementhalters ausübt. Durch die Federkraft wird das Testelement zusätzlich in Position gehalten.

Das Führungselement kann bei der vorliegenden Erfindung auf der Seite, auf der ein Testelement in den Testelementhalter eingeführt wird, eine rampen- oder trichterförmige Einführöffnung aufweisen. Die rampen- oder trichterförmige Einführöffnung erleichtert das Einführen eines Testelements in das Führungselement.

In einer Ausführungsform der vorliegenden Erfindung ist das Führungselement so geformt, dass es eine definierte Verformung eines in den Testelementhalter eingeführten Testelements zur Fixierung während des Gebrauchs hervorruft. Beispielsweise kann das Führungselement so in Längsrichtung gebogen sein, dass ein Testelement beim Einführen in das Führungselement in Längsrichtung definiert verformt wird und in der Messposition unter Biegespannung steht. Dadurch wird der definierte Abstand des Testfelds von der die Analyseeinheit und die Detektionseinheit umfassenden Messeinheit sichergestellt. Ebenso könnte das als zwei Nuten ausgebildete Führungselement so zur Einführebene des Testelements geneigt sein, dass das Testelement in dem Führungselement definiert in Querrichtung verformt wird. Dadurch wird ebenfalls eine Fixierung des Testelements in dem Testelementhalter erreicht.

Die Erfindung bezieht sich weiterhin auf die Verwendung des erfindungsgemäßen Analysesystems zur Analyse des Glukosegehalts in Blut auf einem streifenförmigen Testelement.

Anhand der Zeichnung wird die Erfindung nachstehend näher erläutert.

Es zeigt:
- Figur 1A: das Einführen eines Testelements in ein Führungselement eines Analysesystems aus dem Stand der Technik,
- Figur 1B: die Probenaufgabe auf ein Testelement in einem Führungselement eines Analysesystems aus dem Stand der Technik,
- Figur 1C: die Entnahme eines Testelements nach einer Messung aus einem Führungselement eines Analysesystems aus dem Stand der Technik,
- Figur 2A: den Ablauf einer manuellen Handhabung eines Testelements in einem Analysesystem aus dem Stand der Technik,
- Figur 2B: den Ablauf eines automatischen Transports eines Testelements in einem Analysesystem aus dem Stand der Technik,
- Figur 3A: die schematische ausschnittsweise Darstellung eines erfindungsgemäßen Analysesystems, in das ein Testelement eingeführt wird,
- Figur 3B: die Probenaufgabe auf ein Testelement in dem Führungselement eines erfindungsgemäßen Analysesystems,
- Figur 3C: die Entnahme eines Testelements nach einer Messung aus einem Führungselement eines erfindungsgemäßen Analysesystems,
- Figur 4: ein Testelement mit einer Verjüngung im Bereich des Probenaufgabeorts,
- Figur 5: einen mehrteiligen Testelementhalter mit Druckfedern in einem erfindungsgemäßen Analysesystem und
- Figur 6: einen mehrteiligen Testelementhalter mit schrägen Führungsflächen.

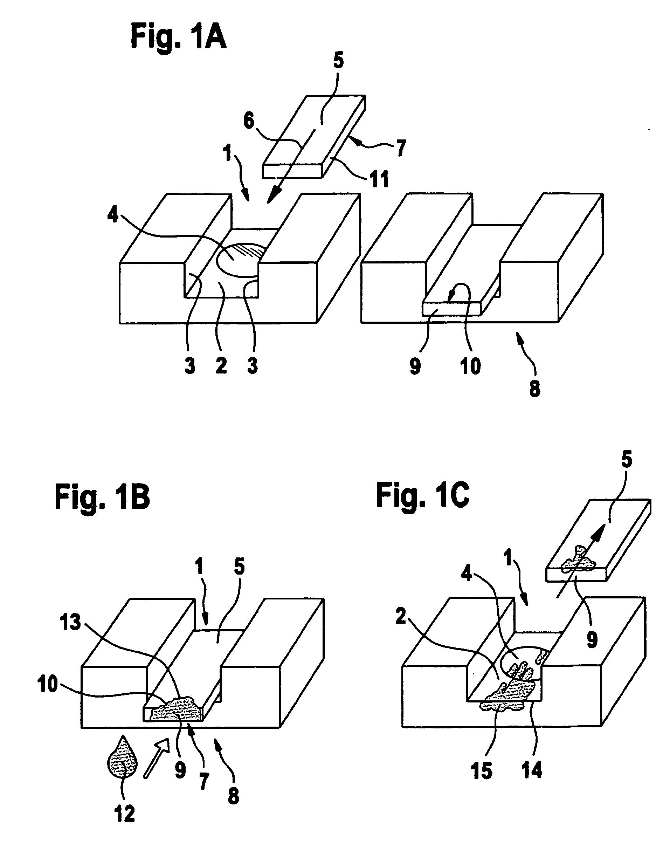

Figur 1A zeigt schematisch das Einführen eines Testelements in ein Führungselement eines Analysesystems aus dem Stand der Technik.

Dabei ist das Führungselement 1 eine Art offener Graben mit einer Auflagefläche 2 und Seitenwänden 3. In der Auflagefläche 2 befindet sich ein Optikfenster 4 (symbolisiert durch den Kreis), unter dem eine (nicht dargestellte) Messoptik zur photometrischen Auswertung des Testelements 5 angeordnet ist. Ein Testelement 5 wird in Einführrichtung 6 in das Führungselement 1 eingeführt und gleitet dabei über seine gesamte Breite mit seiner Unterseite 7 über die Auflagefläche 2. Geführt wird es beim Einführen durch die Seitenwände 3, an denen die Seitenflächen 11 des Testelements 5 entlanggleiten. In der Probenaufgabeposition 8 liegt das Testelement 5 großflächig auf der Auflagefläche 2 auf. Das Testelement 5 ragt mit seinem Ende 9, das den Probenaufgabeort 10 enthält, über die Auflagefläche 2 hinaus.

Dieses in Figur 1A dargestellte Design des Führungselements 1, in welchem das nur seitlich geführte Testelement 5 mit der Unterseite 7 flächig aufliegt, ist dafür konzipiert, dass das Testelement nach der Messung (und dem damit verbundenen Auftrag der Probe) nicht mit dem mit Probe beaufschlagten Ende durch das Führungselement 1 geführt wird. In einem Analysesystem mit einer Remagazinierungsfunktion findet aber genau diese Bewegung statt. Dies wird in Figuren 1B und 1C demonstriert.

Figur 1B zeigt die Probenaufgabe auf ein Testelement in einem Führungselement eines Analysesystems aus dem Stand der Technik.

Das Testelement 5 wird, geführt durch das Führungselement 1, in die Probenaufgabeposition 8 geschoben, wie in Figur 1A dargestellt. Zum Auftrag der Probe 12, beispielsweise von Blut, steht das Testelement 5 in dieser Position etwas aus dem Analysesystem heraus. Es wird Probe 12 am Probenaufgabeort 10 auf das Testelement 5 aufgetragen. Dabei taucht das Ende 9 des Testelements 5 etwas in die Probe 12 hinein, so dass es auf seiner Oberseite 13 und Unterseite 7 mit der flüssigen Probe 12 benetzt wird.

Figur 1C zeigt die Entnahme des Testelements nach einer Messung aus einem Führungselement eines Analysesystems aus dem Stand der Technik.

Wird das Testelement 5 nach der Messung entgegen der Einführrichtung 6 (z.B. zum Remagazinieren des Testelements 5) aus der Probenaufgabeposition 8 durch das Führungselement 1 zurückgezogen, so werden an dem Ende 9 des Testelements 5 haftende Tropfen der Probe 12 an der Kante 14 des Führungselements 1 abgestreift. Durch Kapillarkräfte wird Probenmaterial in den Spalt zwischen dem Testelement 5 und der Auflagefläche 2 gezogen und auch durch das Herausziehen des Testelements 5 über die Auflagefläche 2 weiter über diese verteilt. Es entsteht eine Verschmutzung 15 eines weiten Bereichs der Auflagefläche 2, inklusive des Optikfensters 4 mit Probenmaterial. Ein weiterer Nachteil des in Figuren 1A bis 1C gezeigten Führungselements 1 eines Analysesystems aus dem Stand der Technik ist, dass das Optikfenster 4 versenkt in die Auflagefläche 2 für das Testelement 5 eingebaut werden muss, um es vor einer Beschädigung durch die Reibung des Testelements 5 beim Einführen und bei der Entnahme des Testelements 5 aus dem Führungselement 1 zu schützen.

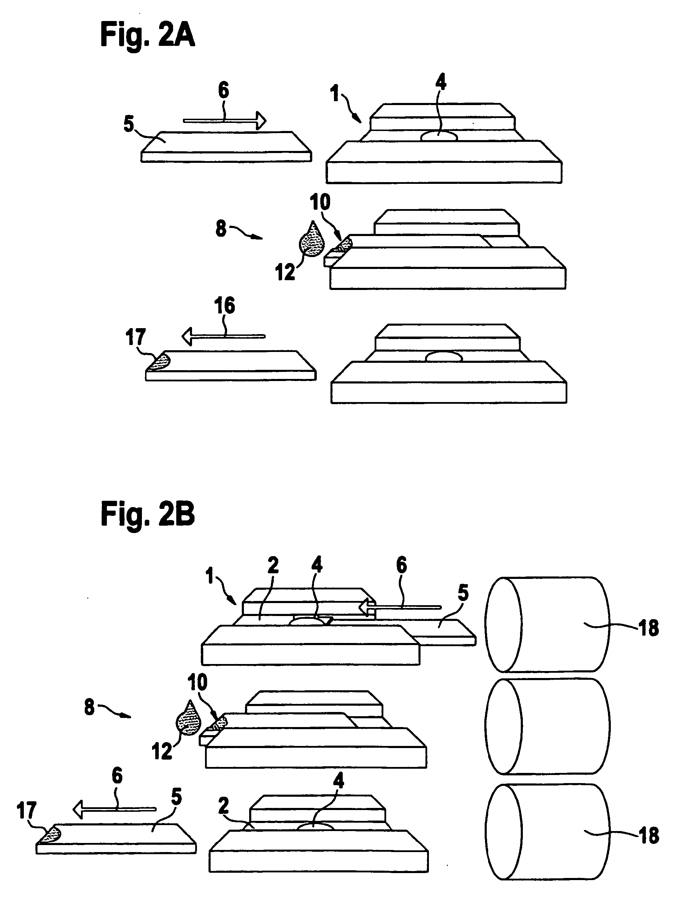

Figuren 2A und 2B demonstrieren, wie Testelemente im Stand der Technik manuell oder automatisch gehandhabt werden. Figur 2A zeigt den Ablauf einer manuellen Handhabung eines Testelements in einem Analysesystem aus dem Stand der Technik.

Bei manuell bedienten Analysesystemen wird das Testelement 5 vom Anwender in Einführrichtung 6 in das Führungselement 1 über das Optikfenster 4 in das Analysesystem geschoben. In der Probenaufgabeposition 8 wird Probe 12 auf den Probenaufgabeort 10 gegeben und anschließend die Messung durchgeführt. Nach der Messung wird das Testelement 5 aus dem Analysesystem durch den Bediener herausgezogen. Das Herausziehen erfolgt in der Entnahmerichtung 16, die der Einführrichtung 6 entgegengesetzt ist. Die mit Probe 12 benetzte Kante 17 des Testelements 5 berührt so nie die Auflagefläche 2 und das Optikfenster 4, so dass eine Verschmutzung vermieden wird. Eine Entnahme in derselben Richtung wie die Einführrichtung 6 ist bei solchen Analysesystemen im Stand der Technik nicht vorgesehen und würde zu einer Verschmutzung der Auflagefläche 2 führen, wie bezüglich Figur 1C beschrieben.

Figur 2B zeigt den Ablauf eines automatischen Transports eines Testelements in einem Analysesystem aus dem Stand der Technik.

Bei automatisch bedienten Analysesystemen aus dem Stand der Technik wird das Testelement 5 aus einem Vorratsmagazin 18 (z.B. durch einen nicht dargestellten Stößel) in der Einführrichtung 6 in das Führungselement 1 geschoben. In der Probenaufgabeposition 8 wird Probe 12 an dem Probenaufgabeort 10 auf das Testelement 5 aufgetragen und eine Messung durchgeführt. Nach der Messung wird das Testelement 5 (z.B. von dem Stößel) aus dem Führungselement 1 in derselben Richtung wie die Einführrichtung 6 herausgestoßen. Die mit Probe 12 benetzte Kante 17 des Testelements 5 berührt daher nicht die Auflagefläche 2 oder das Optikfenster 4, so dass eine Verschmutzung mit Probe 12 vermieden wird. Eine Entnahme des Testelements nach der Messung in der der Einführrichtung 6 entgegengesetzten Richtung (z.B. zur Wiederaufnahme in das Vorratsmagazin 18) ist bei solchen Analysesystemen im Stand der Technik nicht vorgesehen und würde zu einer Verschmutzung der Auflagefläche 2 führen, wie bezüglich Figur 1C beschrieben.

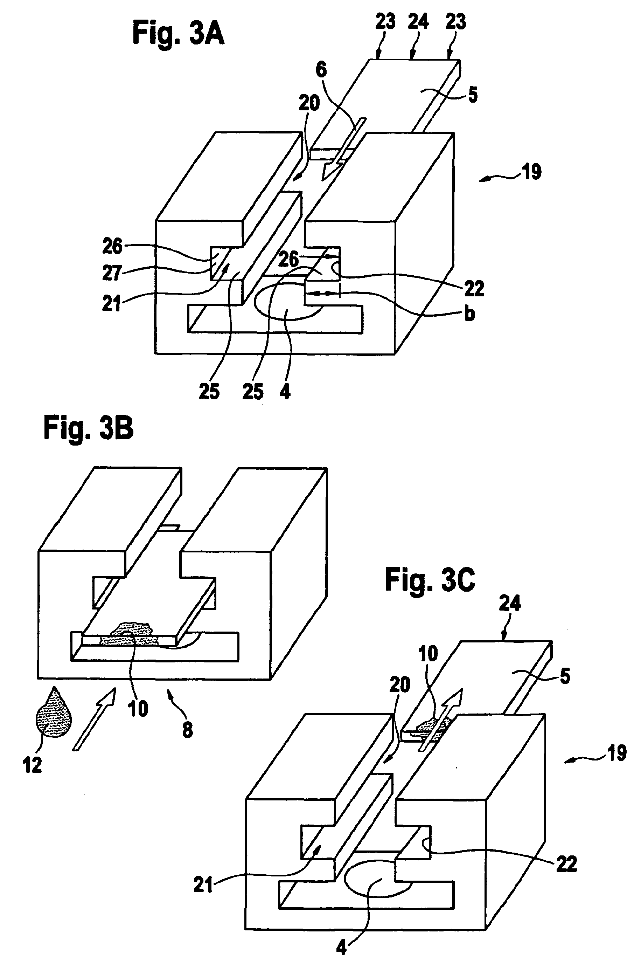

Figur 3A zeigt die schematische Darstellung eines Ausschnitts aus einem erfindungsgemäßen Analysesystem, in das ein Testelement eingeführt wird.

Das erfindungsgemäße Analysesystem enthält eine Analyseeinheit und eine Detektionseinheit zur photometrischen Analyse, die beide unter einem Optikfenster 4 in Figur 3A nicht sichtbar angeordnet sind. Das Analysesystem umfasst einen Testelementhalter 19, in den ein Testelement 5 reversibel eingeführt und in dem es relativ zu den unter dem Optikfenster 4 angeordneten Analyse- und Detektionseinheiten positioniert werden kann. Der Testelementhalter 19 enthält ein Führungselement 20, das zur lateralen Führung des Testelements geeignet ist. Das Führungselement 20 umfasst zwei sich gegenüberliegende Nuten 21, 22, in die das Testelement 5 in seinem äußeren Bereich 23 eingeschoben werden kann. Das Testelement 5 wird dabei in dem Testelementhalter 19 ausschließlich in seinem äußeren Bereich 23 gehalten und geführt und ein innerer Bereich 24 des in den Testelementhalter 19 eingeführten Testelements 5 bleibt freiliegend. Die Nuten 21, 22 umschließen den äußeren Bereich 23 des eingeführten Testelements 5 derart, das es weder nach oben noch nach unten aus dem Führungselement 20 herausfallen kann (geschlossene Führung). Das Führungselement 20 weist Trageflächen 25 auf, auf denen das Testelement 5 mit Auflageflächen in seinem äußeren Bereich 23 aufliegen kann, und Führungsflächen 26, an denen das Testelement 5 beim Transport entlang geführt wird, auf. Um den äußeren Bereich 23 des Testelements 5 verbleibt beim Einführen genügend Spiel, um das Testelement 5 mit geringem Kraftaufwand in dem Führungselement 20 bewegen zu können und den Verschleiß sowohl des Testelements 5 als auch des Führungselements 20 gering zu halten. Die Trageflächen 25 weisen eine Breite b zwischen 0,1 mm und 1 mm auf.

Das Testelement 5 wird in Einführrichtung 6 in das Führungselement 20 eingeführt. Das Führungselement 20 ist oberhalb des die Analyse- und Detektionseinheit abdeckenden Optikfensters 4 in dem Analysesystem angeordnet. Ein in das Führungselement 20 eingeführtes Testelement 5 weist in jeder Position mindestens einen Abstand von 1 mm zu der Messoptik auf. Dieser Abstand gewährleistet, dass kein Probenmaterial von dem Probenaufgabeort 10 des Testelements 5 durch Kapillarkräfte in den Spalt zwischen dem Testelement 5 und dem Optikfenster 4 gezogen wird.

Der Testelementhalter 19 kann einen (nicht dargestellten) Anschlag enthalten. Beispielsweise kann das eine Ende 27 der Nuten 21, 22 verschlossen sein und so als Anschlag dienen. Beim Einführen in das Führungselement 20 stößt dann ein Testelement 5 an den Anschlag, sobald es seine Probenaufgabeposition erreicht hat. Ein solcher Anschlag kann auch für Testelemente 5 genutzt werden, die aus dem Analysesystem zur Probenaufgabe herausstehen sollen, wenn die Testelemente 5 eine entsprechende Form aufweisen, insbesondere, wenn sie im Bereich des Probenaufgabeortes verjüngt sind, z.B. in Form einer Schulter oder eines Rückschnitts. Des Weiteren kann das Führungselement 20 (nicht dargestellte) Positionsschalter enthalten, die eine genaue Positionierung des Testelements 5 erlauben.

In dem Testelementhalter 19 kann das Führungselement 20 so gestaltet sein, dass das Testelement 5 durch Reibung oder durch (nicht dargestellte) integrierte Halteklammern oder Druckfedern in bestimmten Positionen, insbesondere in der Probenaufgabe- und der Messposition, fixiert wird. Alternativ oder zusätzlich dazu ist eine Fixierung des Testelements 5 mit Hilfe des Antriebselements (Stößel, Haken, Klammer...) möglich, das zum automatischen Transport des Testelements 5 in dem erfindungsgemäßen Analysesystem dient.

Figur 3B zeigt die Probenaufgabe auf ein Testelement in dem Führungselement eines erfindungsgemäßen Analysesystems.

Das Testelement befindet sich in der Probenaufgabeposition 8, in der die Probe 12 auf den Probenaufgabeort 10 des Testelements 5 gegeben wird. Die anschließende Messung kann ebenfalls in der Probenaufgabeposition 8 stattfinden oder in einer speziellen Messposition in dem Analysesystem durchgeführt werden.

Figur 3C zeigt die Entnahme eines Testelements nach einer Messung aus einem Führungselement eines erfindungsgemäßen Analysesystems.

Nach dem Messvorgang wird das Testelement 5 durch das Führungselement 20 hindurch aus dem Testelementhalter 19 entnommen und gegebenenfalls in einem (nicht dargestellten) Vorratsmagazin abgelegt (Remagazinierung). Der mit Probenmaterial benetzte innere Bereich 24 des Testelements 5 in der Umgebung des Probenaufgabeorts 10 wird dabei in sicherem Abstand zu dem Optikfenster 4 und den Nuten 21, 22 durch den Testelementhalter geführt. Es wird somit kein überschüssiges Probenmaterial abgestreift, das eine Verschmutzung des Inneren des Analysesystems hervorrufen würde. Das Optikfenster 4 wird ferner nicht mechanisch durch das verschobene Testelement belastet, so dass es nicht versenkt eingebaut werden muss, und sich dadurch das Design des Analysesystems vereinfacht. Die unter dem Optikfenster 4 vorhandene Messoptik ist auf ihren Abstand zum Testelement 5 angepasst.

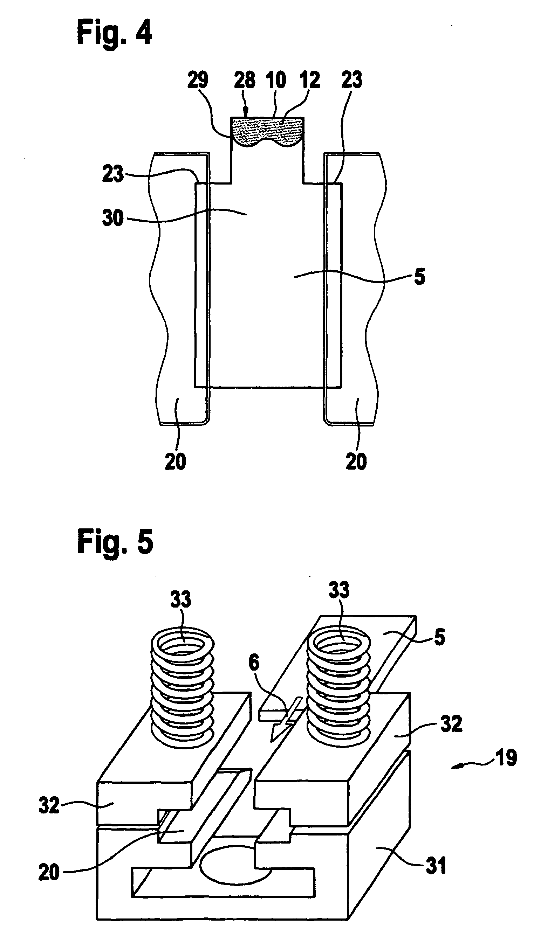

Figur 4 zeigt ein Testelement mit einer Verjüngung im Bereich des Probenaufgabeorts.

Als eine weitere Maßnahme gegen eine Benetzung des Testelements 5 mit Probe 12 über seine gesamte Breite und gegen eine dadurch möglicherweise hervorgerufene Verschmutzung des Führungselements des erfindungsgemäßen Analysesystems mit Probenmaterial, ist das Testelement an einem Ende 28 im Bereich des Probenaufgabeorts 10 verjüngt. Die Verjüngung 29 hat die Form eines Rückschnitts, der so gewählt ist, dass bei maximaler Ausbreitung der Probe 12 der breite Bereich 30 des Testelements 5 nicht durch die Probe 12 benetzt wird. So wird eine Verunreinigung des Führungselements 20, das nur mit dem äußeren Bereich 23 des Testelements 5 in Berührung kommt, verhindert.

Figur 5 zeigt einen zweiteiligen Testelementhalter 19 mit Druckfedern in einem erfindungsgemäßen Analysesystem.

Der Testelementhalter 19 umfasst einen unteren Teil 31 und einen oberen Teil 32, die übereinander liegen und seitlich gegen ein Verschieben gesichert sind. An dem oberen Teil 32 greifen zwei Druckfedern 33 an, die auf den oberen Teil 32 einer Kraft in Richtung des unteren Teils 31 ausüben. In ein Führungselement 20 zwischen dem unteren Teil 31 und dem oberen Teil 32 kann ein Testelement 5 in Einführrichtung 6 eingeschoben werden und die beiden Teile 31, 32 werden dann durch das Testelement 5 auseinandergedrückt und das Testelement 5 passt spielfrei in das Führungselement 20. Durch den Druck der Druckfedern 33 kann das Testelement 5 in einer gewünschten Position in dem Führungselement 20 zusätzlich fixiert werden.

Auf der Seite, auf der ein Testelement 5 in den Testelementhalter 19 eingeführt werden kann (in Figur 5 die nicht dargestellte Rückseite), weist der Testelementhalter 19 vorzugsweise eine rampen- oder trichterförmige Einführöffnung auf, durch die das Testelement 5 in das Führungselement 20 geschoben werden kann. Diese Einführöffnung erleichtert das Einführen eines Testelements 5, das höher als die Innenhöhe des Führungselements 20 vor dem Einführen des Testelements 5 ist.

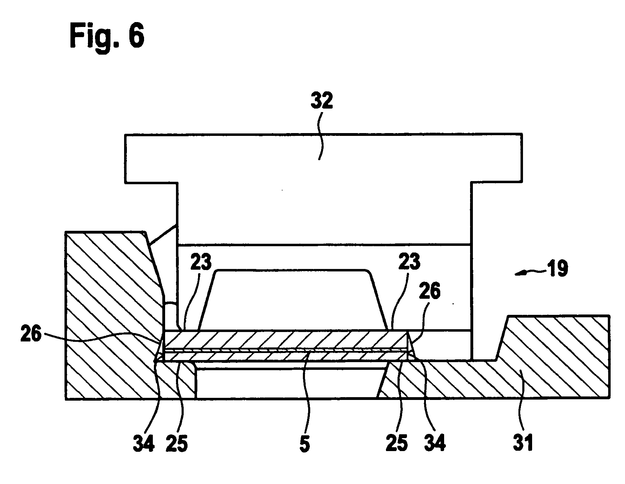

Figur 6 zeigt einen zweiteiligen Testelementhalter mit schrägen Führungsflächen.

Der Testelementhalter 19 umfasst einen unteren Teil 31 und einen oberen Teil 32, zwischen die ein Testelement 5 in ein Führungselement 20 eingeschoben werden kann. Das Führungselement 20 hat Trageflächen 25, auf denen das Testelement 5 mit Auflageflächen in seinem äußeren Bereich 23 aufliegt, und Führungsflächen 26, an denen die Seitenflächen 34 des Testelements entlanggeführt werden. Die Führungsflächen 26 sind dabei schräg in Bezug auf die Seitenflächen 34 des Testelements 5 angeordnet, um eine Verschmutzung der Führungsflächen 26 durch die Seitenflächen 34 (z.B. durch daran haftenden Klebstoff) zu vermeiden.

### Bezugszeichenliste

- 1: Führungselement (Stand der Technik)
- 2: Auflagefläche
- 3: Seitenwände
- 4: Optikfenster
- 5: Testelement
- 6: Einführrichtung
- 7: Unterseite des Testelements
- 8: Probenaufgabeposition
- 9: Ende des Testelements
- 10: Probenaufgabeort
- 11: Seitenflächen des Testelements
- 12: Probe
- 13: Oberseite des Testelements
- 14: Kante des Führungselements
- 15: Verschmutzung
- 16: Entnahmerichtung
- 17: Kante des Testelements
- 18: Vorratsmagazin
- 19: Testelementhalter
- 20: Führungselement
- 21: erste Nut
- 22: zweite Nut
- 23: äußerer Bereich des Testelements
- 24: innerer Bereich des Testelements
- 25: Trageflächen des Führungselements
- 26: Führungsflächen des Führungselements
- 27: Ende der Nuten
- 28: Ende des Testelements
- 29: Verjüngung
- 30: breiter Bereich
- 31: unterer Teil des Testelementhalters
- 32: oberer Teil des Testelementhalters
- 33: Druckfedern
- 34: Seitenflächen des Testelements

## Patentansprüche

1. Analysesystem zur Analyse einer Probe (12) auf einem Testelement (5), umfassend
- eine Analyseeinheit zu Generierung eines Signals in Abhängigkeit von einem in der Probe (12) enthaltenen Analyten und
- eine Detektionseinheit zur Detektion des Signals,
- einen Testelementhalter (19), in den das Testelement (5) reversibel eingeführt und in dem es relativ zu der Analyseeinheit und zu der Detektionseinheit positioniert werden kann,
wobei das Testelement (5) ein streifenförmiges Testelement (5) ist, wobei der Testelementhalter (19) mindestens ein Führungselement (20) enthält, das zur lateralen Führung des Testelements (5) geeignet ist, so dass das Testelement (5) in dem Testelementhalter (19) ausschließlich an einem äußeren Bereich (23) des Testelements (5) gehalten und geführt wird und ein innerer Bereich (24) des in den Testelementhalter (19) eingeführten Testelements (5) freiliegend bleibt, wobei der innere Bereich (24) ein mittlerer Teil beider Oberflächen des Testelements (5) ist, wobei das Testelement (5) einen Probenaufgabeort (10) in dem inneren Bereich (24) enthält, wobei das Führungselement (20) zwei sich gegenüber liegende Nuten (21, 22) enthält, in die das Testelement (5) mit seinem äußeren Bereich (23) eingeschoben werden kann.

2. Analysesystem gemäß Anspruch 1, **dadurch gekennzeichnet, dass** das Führungselement (20) Trageflächen (25), auf denen das Testelement (5) mit Auflageflächen in seinem äußeren Bereich (23) aufliegt, und Führungsflächen (26), an denen Seitenflächen (34) des Testelements (5) entlang geführt werden, enthält.

3. Analysesystem gemäß Anspruch 2, **dadurch gekennzeichnet, dass** die Führungsflächen (26) schräg in Bezug auf die Seitenflächen (34) des Testelements (5) angeordnet sind.

4. Analysesystem gemäß einem der Ansprüche 2 oder 3, **dadurch gekennzeichnet, dass** die Trageflächen (25) eine Breite von 0,1 mm bis 1 mm aufweisen.

5. Analysesystem gemäß einem der Ansprüche 1 bis 4, **dadurch gekennzeichnet, dass** die Analyseeinheit und die Detektionseinheit Teile einer Messoptik sind, die zur photometrischen Auswertung des Testelements (5) dient.

6. Analysesystem gemäß einem der Ansprüche 1 bis 5, **dadurch gekennzeichnet, dass** das Führungselement (20) oberhalb der Analyseeinheit und/oder der Detektionseinheit in dem Analysesystem angeordnet ist.

7. Analysesystem gemäß einem der Ansprüche 1 bis 6, bei dem das Führungselement (20) so angeordnet ist, dass ein in das Führungselement (20) eingeführtes Testelement (5) in jeder Position mindestens einen Abstand von 1 mm zu der Analyseeinheit und zu der Detektionseinheit aufweist.

8. Analysesystem gemäß einem der Ansprüche 1 bis 7, **dadurch gekennzeichnet, dass** der Testelementhalter (19) einen Anschlag enthält, gegen den ein Testelement (5) beim Einführen in das Führungselement (20) anschlägt, sobald eine definierte Position des Testelements (5) in dem Testelementhalter (19) erreicht wird.

9. Analysesystem gemäß einem der vorhergehenden Ansprüche, das einen Vorratsbehälter für eine Vielzahl von Testelementen (5) umfasst, in den die Testelemente (5) nach Gebrauch aus dem Testelementhalter (19) zurücktransportiert werden.

10. Analysesystem gemäß dem vorhergehenden Anspruch mit einer Transporteinrichtung zur automatischen Entnahme eines Testelements (5) aus dem Vorratsbehälter, zum automatischen Transport des Testelements (5) in den Testelementhalter (19) und zum automatischen Rücktransport des Testelements (5) nach Gebrauch in den Vorratsbehälter.

11. Analysesystem gemäß einem der vorhergehenden Ansprüche, **dadurch gekennzeichnet, dass** der Testelementhalter (19) mindestens zweiteilig ist, wobei ein in den Testelementhalter (19) eingeführtes Testelement (5) in seinem äußeren Bereich (23) auf einem unteren Teil (31) des Testelementhalters (19) aufliegt und ein separater oberer Teil (32) des Testelementhalters (19) auf dem Testelement (5) in seinem äußeren Bereich (23) aufliegt.

12. Analysesystem gemäß dem vorhergehenden Anspruch, **dadurch gekennzeichnet, dass** an dem oberen Teil (32) des Testelementhalters (19) mindestens eine Druckfeder (32) angeordnet ist, die auf den oberen Teil (32) eine Kraft in Richtung des unteren Teils (31) des Testelementhalters (19) ausübt.

13. Analysesystem gemäß einem der vorhergehenden Ansprüche, **dadurch gekennzeichnet, dass** das Führungselement (20) auf der Seite, auf der ein Testelement (5) in den Testelementhalter (19) eingeführt wird, eine rampen- oder trichterförmige Einführöffnung aufweist.

14. Analysesystem gemäß einem der vorhergehenden Ansprüche, weiterhin umfassend ein Testelement (5).

15. Analysesystem nach dem vorhergehenden Anspruch, **dadurch gekennzeichnet, dass** das Testelement (5) an einem Ende in dem inneren Bereich (24) einen Probenaufgabeort (10) umfasst, wobei das Testelement (5) im Bereich des Probenaufgabeorts (10) verjüngt ist.

16. Analysesystem nach einem der beiden vorhergehenden Ansprüche, **dadurch gekennzeichnet, dass** das Testelement (5) ein Testfeld aufweist, in dem die Probe (12) analysiert wird, das in dem inneren Bereich (24) des Testelements (5) positioniert ist.

17. Analysesystem nach dem vorhergehenden Anspruch, **dadurch gekennzeichnet, dass** das Testelement (5) eine Kapillare zum Leiten der Probe (12) zu dem Testfeld enthält.

18. Analysesystem gemäß einem der fünf vorhergehenden Ansprüche, **dadurch gekennzeichnet, dass** das Führungselement (20) so geformt ist, dass es eine definierte Verformung eines in den Testelementhalter (19) eingeführten Testelements (5) zur Fixierung während des Gebrauchs hervorruft.

19. Verwendung eines Analysesystems gemäß einem der vorhergehenden Ansprüche zur Analyse des Glucose-Gehalts in Blut auf einem streifenförmigen Testelement.

## Claims

1. Analysis system for analyzing a sample (12) on a test element (5),
comprising
- an analysis unit for generating a signal as a function of an analyte contained in the sample (12), and
- a detection unit for detecting the signal,
- a test element holder (19) into which the test element (5) can be reversibly introduced
and in which it can be positioned relative to the analysis unit and the detection unit, the test element (5) being a strip-shaped test element (5), the test element holder (19) containing at least one guide element (20) which is suitable for laterally guiding the test element (5), so that the test element (5) in the test element holder (19) is held and guided only on an outer region (23) of the test element (5), and an inner region (24) of the test element (5) introduced into the test element holder (19) remains free, the inner region (24) being a central part of the two surfaces of the test element (5), the test element (5) containing a sample application site (10) in the inner region (24), the guide element (20) containing two mutually opposite grooves (21, 22), into which the test element (5) can be inserted with its outer region (23).

2. Analysis system according to Claim 1, **characterized in that** the guide element (20) contains support faces (25), on which the test element (5) rests via bearing faces in its outer region (23), and guide faces (26) along which side faces (34) of the test element (5) are guided.

3. Analysis system according to Claim 2, **characterized in that** the guide faces (26) are arranged obliquely with respect to the side faces (34) of the test element (5).

4. Analysis system according to Claim 2 or 3, **characterized in that** the support faces (25) have a width of from 0.1 mm to 1 mm.

5. Analysis system according to one of Claims I to 4, **characterized in that** the analysis unit and the detection unit are parts of measuring optics which are used to photometrically evaluate the test element (5).

6. Analysis system according to one of Claims I to 5, **characterized in that** the guide element (20) is arranged above the analysis unit and/or the detection unit in the analysis system.

7. Analysis system according to one of Claims 1 to 6, **characterized in that** the guide element (20) is arranged so that a test element (5) introduced into the guide element (20) is at a distance of at least 1 mm from the analysis unit and the detection unit in any position.

8. Analysis system according to one of Claims 1 to 7, **characterized in that** the test element holder (19) has a stop, against which a test element (5) abuts when it is being introduced into the guide element (20) as soon as a defined position of the test element (5) in the test element holder (19) is reached.

9. Analysis system according to one of the preceding claims, which comprises a storage container for a multiplicity of test elements (5), into which the test elements (5) are transported back from the test element holder (19) after use.

10. Analysis system according to the preceding claim, having a transport device for automatically extracting a test element (5) from the storage container, for automatically transporting the test element (5) into the test element holder (19) and for automatically transporting the test element (5) back into the storage container after use.

11. Analysis system according to one of the preceding claims, **characterized in that** the test element holder (19) is made of at least two parts, with a test element (5) introduced into the test element holder (19) resting in its outer region (23) on a lower part (31) of the test element holder (19) and with a separate upper part (32) of the test element holder (19) resting on the test element (5) in its outer region (23).

12. Analysis system according to the preceding claim, **characterized in that** at least one pressure spring (33), which exerts a force on the upper part (32) in the direction of the lower part (31) of the test element holder (19), is arranged on the upper part (32) of the test element holder (19).

13. Analysis system according to one of the preceding claims, **characterized in that** the guide element (20) has a ramp- or funnel-shaped introduction opening on the side where a test element (5) is introduced into the test element holder (19).

14. Analysis system according to one of the preceding claims, further comprising a test element (5).

15. Analysis system according to the preceding claim, **characterized in that** the test element (5) comprises a sample application site (10) at one end in the inner region (24), the test element (5) being tapered in the region of the sample application site (10).

16. Analysis system according to either of the two preceding claims, **characterized in that** the test element (5) has a test field where the sample (12) is analyzed, which is positioned in the inner region (24) of the test element (5).

17. Analysis system according to the preceding claim, **characterized in that** the test element (5) contains a capillary for delivering the sample (12) to the test field.

18. Analysis system according to one of the five preceding claims, **characterized in that** the guide element (20) is shaped so that it causes a defined deformation of a test element (5) as it is being introduced into the test element holder (19), in order to fix it during use.

19. Use of an analysis system according to one of the preceding claims for analyzing the glucose content in blood on a strip-shaped test element.

## Revendications

1. Système d'analyse pour analyser un échantillon (12) sur un élément de test (5), comprenant
- une unité d'analyse pour la génération d'un signal en fonction d'une analyte contenue dans l'échantillon (12) et
- une unité de détection pour la détection du signal,
- un support pour l'élément de test (19), dans lequel l'élément de test (5) peut être inséré de façon réversible et dans lequel il peut être positionné par rapport à l'unité d'analyse et à l'unité de détection,
l'élément de test (5) étant un élément de test (5) en forme de bande, le support d'élément de test (19) comportant au moins un élément guide (20) qui est adapté pour le guidage latéral de l'élément de test (5), de sorte que dans le support d'élément de test (19) l'élément de test (5) soit exclusivement maintenu et guidé sur une zone extérieure (23) de l'élément de test (5) et qu'une zone intérieure (24) de l'élément de test (5) introduite dans le support d'élément de test (19) reste à découvert, la zone intérieure (24) étant une partie centrale des deux surfaces de l'élément de test (5), l'élément de test (5) comportant dans la zone intérieure (24) un point de charge de l'échantillon (10), l'élément guide (20) comportant deux rainures (21, 22) opposées dans lesquelles l'élément de test (5) peut être inséré par sa zone extérieure (23).

2. Système d'analyse selon la revendication 1, **caractérisé en ce que** l'élément guide (20) comporte des surfaces porteuses (25), sur lesquelles l'élément de test (5) s'appuie par des surfaces d'appui sur sa zone extérieure (23), et des surfaces de guidage (26), le long desquelles des surfaces latérales (34) de l'élément de test (5) sont guidées.

3. Système d'analyse selon la revendication 2, **caractérisé en ce que** les surfaces de guidage (26) sont placées en inclinaison par rapport aux surfaces latérales (34) de l'élément de test (5).

4. Système d'analyse selon l'une quelconque des revendications 2 ou 3, **caractérisé en ce que** les surfaces porteuses (25) ont une largeur de 0,1 mm à 1 mm.

5. Système d'analyse selon l'une quelconque des revendications 1 à 4, **caractérisé en ce que** l'unité d'analyse et l'unité de détection sont des pièces d'un système optique de mesure, qui sert à l'évaluation photométrique de l'élément de test (5).

6. Système d'analyse selon l'une quelconque des revendications 1 à 5, **caractérisé en ce que** l'élément guide (20) est placé au-dessus de l'unité d'analyse et/ou de l'unité de détection dans le système d'analyse.

7. Système d'analyse selon l'une quelconque des revendications 1 à 6, dans lequel l'élément guide (20) est placé de telle sorte que dans chaque position, un élément de test (5) introduit dans l'élément guide (20) présente au moins un écart de 1 mm par rapport à l'unité d'analyse et à l'unité de détection.

8. Système d'analyse selon l'une quelconque des revendications 1 à 7, **caractérisé en ce que** le support d'élément de test (19) comporte une butée contre laquelle un élément de test (5) vient buter lors de l'introduction dans l'élément guide (20) dès qu'une position définie de l'élément de test (5) dans le support d'élément de test (19) est atteinte.

9. Système d'analyse selon l'une quelconque des revendications précédentes, qui comprend un réservoir de stockage pour une pluralité d'éléments de test (5), dans lequel les éléments de test (5) sont retransportés à partir du support d'élément de test (19), après utilisation.

10. Système d'analyse selon la revendication précédente, avec un dispositif de transport pour le prélèvement automatique d'un élément de test (5) dans le réservoir de stockage, pour le transport automatique de l'élément de test (5) dans le support d'élément de test (19) et pour le transport en retour automatique de l'élément de test (5) dans le réservoir de stockage, après utilisation.

11. Système d'analyse selon l'une quelconque des revendications précédentes, **caractérisé en ce que** le support d'élément de test (19) est au moins en deux parties, un élément de test (5) introduit dans le support d'élément de test (19) s'appuyant dans sa zone extérieure (23) sur une partie inférieure (31) du support d'élément de test (19) et une partie supérieure (32) séparée du support d'élément de test (19) s'appuyant sur l'élément de test (5) dans sa zone extérieure (23).

12. Système d'analyse selon la revendication précédente, **caractérisé en ce que** sur la partie supérieure (32) du support d'élément de test (19) est placé au moins un ressort de pression (33) qui exerce sur la partie supérieure (32) une force en direction de la partie inférieure (31) du support d'élément de test (19).

13. Système d'analyse selon l'une quelconque des revendications précédentes, **caractérisé en ce que** sur le côté sur lequel un élément de test (5) est introduit dans le support d'élément de test (19), l'élément guide (20) présente un orifice d'introduction en forme de rampe ou d'entonnoir.

14. Système d'analyse selon l'une quelconque des revendications précédentes, comprenant par ailleurs un élément de test (5).

15. Système d'analyse selon la revendication précédente, **caractérisé en ce que** sur une extrémité dans la zone intérieure (24), l'élément de test (5) comprend un point de charge de l'échantillon (10), l'élément de test (5) se rétrécissant dans la zone du point de charge de l'échantillon (10).

16. Système d'analyse selon l'une quelconque des deux revendications précédentes, **caractérisé en ce que** l'élément de test (5) comporte un champ de test dans lequel l'échantillon (12) est analysé, qui est positionné dans la zone intérieure (24) de l'élément de test (5).

17. Système d'analyse selon la revendication précédente, **caractérisé en ce que** l'élément de test (5) comporte un capillaire pour diriger l'échantillon (12) vers le champ de test.

18. Système d'analyse selon l'une quelconque des cinq revendications précédentes, **caractérisé en ce que** l'élément guide (20) est façonné conformé de manière à provoquer une déformation définie d'un élément de test (5) introduit dans le support d'élément de test (19), pour sa fixation pendant l'utilisation.

19. Utilisation d'un système d'analyse selon l'une quelconque des revendications précédentes pour l'analyse de la teneur en glucose du sang sur un élément de test en forme de bande.
